# EUROPÄISCHE PATENTANMELDUNG

(11) **EP 1 867 955 A1**
(43) Veröffentlichungstag der Anmeldung: **19.12.2007**
(21) Anmeldenummer: 06011818.9
(22) Anmeldetag: 08.06.2006
(51) Int. Cl.: G01D 1/18, A61B 19/00, A61B 5/00, A61B 6/00

(54) **Kalibriertes medizintechnisches Instrument mit Umgebungssensor**

(71) Anmelder: BrainLAB AG, 85551 Kirchheim/Heimstetten (DE)
(72) Erfinder: Goldbach, Günter, 85457 Wörth/Wifling (DE)
(74) Vertreter: Schwabe - Sandmair - Marx

(57) **Zusammenfassung**

Die Erfindung betrifft ein kalibriertes Instrument mit mindestens einem Sensor (1a, 1b, 1c), um Umwelteinflüsse zu detektieren, welche die Kalibrierung oder Funktionsfähigkeit des Instrumentes (2) verändern können, sowie ein Verfahren zum Detektieren mindestens eines externen Einflusses auf ein kalibriertes Instrument (2), wobei mindestens eine externe physikalische oder chemische Größe erfasst und überprüft wird, ob diese Größe die Kalibrierung des Instrumentes (2) verändern kann.

## Beschreibung

Die vorliegende Erfindung bezieht sich auf ein kalibriertes medizinisches oder medizintechnisches Instrument mit mindestens einem Umgebungssensor zum Erfassen oder Messen von Umwelteinflüssen, sowie auf ein Verfahren zum Detektieren von Umwelteinflüssen, welche auf ein kalibriertes Instrument wirken und die Kalibrierung beeinflussen können.

Bei der Verwendung von präkalibrierten oder vorkalibrierten medizinischen oder medizintechnischen Instrumenten oder Systemen, wie zum Beispiel einem präkalibrierten optischen Tracking-System, ist es von großer Bedeutung, dass die bei der Kalibrierung ermittelte technische Spezifikation, wie zum Beispiel die Form oder Geometrie des Instruments oder Systems, seit dem Kalibrier-Vorgang nicht verändert wurde. Weicht bei einem Tracking-System die Ausrichtung einer Kamera zum Beispiel nur im Bereich von einigen Milligrad von der bei der Kalibrierung vorliegenden Ausrichtung ab, so kann es bei der Bilderfassung zum Beispiel schon zu einem Versatz von einem Mikrometer auf dem als Bildsensor dienenden CCD kommen, was je nach Entfernung eines zum Beispiel detektierten Markers bereits zu einer Fehldetektion des Markers im Bereich von einem Millimeter führen kann. Eine solche Fehldetektion führt zu Ungenauigkeiten, welche insbesondere bei chirurgischen Eingriffen schwerwiegende Folgen haben können.

Da präkalibrierte Systeme jedoch verschiedenartigen Umwelteinflüssen ausgesetzt sind, wie zum Beispiel großen Temperaturschwankungen, mechanischen Stössen oder anderen Belastungen kann es aufgrund der Umwelteinflüsse zu einer Veränderung, wie zum Beispiel einer Verformung oder Verbiegung des medizinischen Instrumentes kommen, was zur Folge hat, dass das Instrument nicht mehr kalibriert ist. Zum Beispiel kann es während des Transports eines Instruments oder Systems von einem Hersteller zu einem Kunden oder bei der Bewegung eines mobilen Systems innerhalb eines Gebäudes, wie zum Beispiel eines Krankenhauses, zu Stössen gegen das Instrument kommen, wodurch zum Beispiel die Form, Geometrie oder Ausrichtung von einigen Teilen des Instrumentes verändert wird und somit das Instrument neu kalibriert werden sollte.

Es ist eine Aufgabe der vorliegenden Erfindung sicherzustellen, dass Umwelteinflüsse, welche auf das medizinische Instrument wirken, nicht zu Fehlbehandlungen durch das medizinische Instrument führen.

Diese Aufgabe wird durch ein Instrument und ein Verfahren wie in den unabhängigen Patentansprüchen definiert gelöst. Vorteilhafte Ausführungsformen ergeben sich aus den abhängigen Ansprüchen.

Ein kalibriertes medizinisches oder medizintechnisches Instrument weist erfindungsgemäß mindestens einen und bevorzugt zwei oder mehr Sensoren auf, um Umwelteinflüsse oder äußere Einwirkungen auf das Instrument zu erkennen oder zu detektieren, welche die Form, Kalibrierung oder Geometrie oder allgemein die Funktionsfähigkeit des Instrumentes verändern oder beeinflussen können. Unter einem kalibrierten Instrument wird im Sinne der Erfindung ein Instrument verstanden, dessen für die Funktionsfähigkeit relevante Bestandteile vor dem Einsatz des Instrumentes vermessen oder deren relevante Eigenschaften, wie zum Beispiel die Form und/oder Ausrichtung, bestimmt wurden. Beispielsweise kann bei einem Tracking-System die Positionierung und Ausrichtung der beiden zur Bilderfassung verwendeten Infrarotkameras bestimmt und in einer Datenbank oder als Software hinterlegt werden. Ebenso ist es auch möglich die vollständige Geometrie eines Instrumentes, wie zum Beispiel eines mit Markern verbundenen Pointers zu erfassen, wobei zum Beispiel als relevante für den Einsatz wichtige Bestandteile die Marker und deren relative Position zur Pointer-Spitze angesehen wird. Kommt es zu einer externen Beeinflussung, wie zum Beispiel zur Einwirkung einer externen Kraft auf das Instrument, so kann es zu einer Veränderung des Instrumentes, wie zum Beispiel zu einer Verformung, kommen, welche erfindungsgemäß mittels des mindestens einen Sensors, welcher beispielsweise mit dem Instrument verbunden oder in dieses Instrument integriert sein kann, erfasst werden kann. Somit steht für einen Benutzer ein Indikator zur Verfügung, über welchen ermittelt werden kann, ob zum Beispiel über einen vorgegebenen Grenzwert liegende externe Einflüsse auf das Instrument gewirkt haben, so dass das Instrument möglicherweise nicht mehr kalibriert ist und vor Gebrauch kalibriert werden sollte. Hierdurch kann die spezifizierte Genauigkeit des Systems zuverlässig sichergestellt und somit garantiert werden.

Allgemein kann jedes medizinische oder medizintechnische Instrument erfindungsgemäß mit mindestens einem Sensor versehen oder verbunden werden, um zu überwachen, ob und welche Umwelteinflüsse auf das Instrument einwirken oder eingewirkt haben. Beispielsweise kann ein Sensor an jedem Bestandteil eines Tracking-Systems, insbesondere der Kamera des Tracking-System, einer Instrumenten-Kalibrier-Matrix (ICM; Instrument Calibration Matrix), einem Flouro-Registrierungs-Kit, einem Pointer, einem Ultraschallphantom, einer MR-Spulenanordnung, wie zum Beispiel Kopfspulen, einem Implantat oder allen anderen werkskalibrierten Instrumenten angebracht werden.

Der erfindungsgemäß mit dem Instrument verbundene oder an dem Instrument vorgesehene oder angebrachte mindestens eine Sensor kann ein Temperatur-, Stoss- oder Schock-Sensor, ein Beschleunigungssensor, ein Dehnungssensor, wie zum Beispiel ein Dehnmessstreifen (DMS), ein Feuchtigkeitssensor, ein Magnetfeldsensor, wie zum Beispiel ein Hall-Sensor, ein Drucksensor oder ein Strahlungssensor zur Detektion von UV, IR oder Röntgenstrahlung sein. Es ist auch möglich mehr als einen Sensor an dem Instrument zum Beispiel an verschiedenen Stellen vorzusehen, wobei auch mehrere gleichartige oder verschiedenartige Sensoren an dem Instrument angebracht sein können, um verschiedenartige Umwelteinflüsse, wie zum Beispiel Stöße und Temperaturschwankungen, gleichzeitig zu detektieren.

Vorteilhaft ist der Sensor mit einem Speicher- oder Aufzeichnungsmechanismus verbunden, in welchem die Art oder Stärke der externen auf das Instrument einwirkenden Größe erfasst wird. Optional kann auch ein Timer oder eine Uhr vorgesehen sein, so dass in dem Speicher die Sensor-Signale zusammen mit der Uhrzeit der Erfassung der Sensorsignale abgelegt werden können, um im Falle einer zum Beispiel größeren Einwirkung, wie zum Beispiel eines starken Stoßes auf das Instrument, feststellen zu können, wann sich diese ereignet hat. Weiterhin ist es auch möglich ein Positionserfassungssystem, wie zum Beispiel ein bekanntes GPS-System, an dem medizintechnischen Instrument oder Sensor vorzusehen, so dass auch ermittelt werden kann wo diese Belastungen oder Beeinflussungen des Instrumentes aufgetreten sind, wobei die Positions- oder GPS-Signale auch zusammen mit den Sensorsignalen oder Zeitsignalen in dem Speicher abgelegt werden können.

Zur Stromversorgung kann jede geeignete Energiequelle, wie zum Beispiel eine Batterie, ein Akkumulator oder eine aufladbare Batterie, eine Brennstoffzelle, ein Solarelement oder auch nur ein Kondensator bevorzugt mit hoher Kapazität, wie zum Beispiel ein Gold-Kondensator, vorgesehen sein, welcher den Sensor, falls erforderlich, zusammen mit einer möglicherweise vorhandenen Auswerte- oder Speicher-Elektronik mit Strom versorgt. Je nach Art der zu erfassenden oder messenden Größe können auch Sensoren oder Erfassungsmechanismen verwendet werden, welche keine zusätzliche Stromversorgung benötigen. Zum Beispiel kann die durch Röntgenstrahlung auf das Instrument einwirkende Energie auf speziellen Halbleiterelementen integriert werden oder die Energie zur Aufzeichnung oder Erfassung der externen Einwirkung kann aus dieser Einwirkung selbst gewonnen werden, wobei zum Beispiel die in einem mechanischen Stoss enthaltene Energie zum Beispiel in elektrische Energie umgewandelt werden kann, oder als Energiequelle für eine Erfassungs- oder Aufzeichnungsmechanik dient, wie dies beispielsweise aus Automatik-Armbanduhren bekannt ist. Ebenso können auch piezo-basierte Beschleunigungssensoren verwendet werden, welche mechanische Stöße oder Einwirkungen speichern oder als elektrische Signale weitergeben, wobei der mechanische Stoß in elektrische Energie umgewandelt wird.

Bevorzugt wird an dem Instrument eine Statusanzeige oder die Möglichkeit einer Status-Abfrage vorgesehen, wobei zum Beispiel der Ladezustand der Stromversorgung angezeigt werden kann. Ebenso ist es möglich die zum Beispiel von dem oder den Sensoren erfassten und in dem Speicher abgelegten Einwirkungs-Daten nach Initiierung eines Speicher-Auslesevorganges zum Beispiel durch Betätigung eines Druckknopfes optisch und/oder akustisch auszugeben. Beispielsweise kann eine Infrarot-LED vorgesehen sein, über welche einer Kamera eines Tracking-Systems signalisiert werden kann, dass das im Erfassungsbereich des Tracking-Systems liegende Instrument noch mal kalibriert werden sollte. Ebenso ist es auch möglich ein elektrisches Interface, wie zum Beispiel einen USB-Anschluss, vorzusehen, über welchen der Sensor oder Speicher des Instrumentes angesteuert und zum Beispiel ausgelesen werden kann, um zu ermitteln, ob das Instrument kalibriert werden sollte.

Vorteilhaft kann ein abnehmbarer Datenträger, wie zum Beispiel ein USB-Stick, eine CF-Karte, SD-Karte oder ähnliches vorgesehen sein, welcher zum Beispiel von einem Servicetechniker ausgetauscht und ausgelesen werden kann, so dass die aufgezeichneten Daten als "Logbuch" des Instrumentes abnehmbar oder entnehmbar und zum Beispiel mittels eines geeigneten Service-Rechners ausgelesen und ausgewertet werden können. Vorzugsweise kann das Instrument auch über eine Schnittstelle zur Fernwartung verfügen, so dass beispielsweise über Funk, Infrarot oder andere Datenübertragungsmechanismen ein Auslesen eines in dem Instrument vorgesehenen Speichers oder eine Abfrage der an dem Instrument vorgesehenen Sensoren vorgenommen werden kann.

Gemäß einem weiteren Aspekt bezieht sich die Erfindung auf ein Verfahren zum Detektieren von Umwelteinflüssen oder physikalischen oder chemischen Einwirkungen auf ein kalibriertes medizintechnisches Instrument, um zu ermitteln, ob das Instrument oder Teile davon beeinflusst oder verändert wurden und noch mal kalibriert werden sollten.

Vorzugsweise wird die Erfassung oder Detektion der Umwelteinflüsse kontinuierlich durchgeführt, um sicherzustellen, dass möglichst zu keinem Zeitpunkt unerwünschte und unerkannte Einwirkungen auf das Instrument stattgefunden haben. Die von dem mindestem einen Sensor erfassten Signale werden vorteilhaft aufgezeichnet, wobei beispielsweise ein Zeitstempel zusammen mit den Signalen in einem Speicher abgelegt werden kann, in welchem optional auch Positions- oder GPS-Signale abgelegt werden können.

Bevorzugt wird für mindestens einen oder jeden Sensor ein Grenzwert der zu erfassenden Größe festgelegt, und vorteilhaft ein Warnsignal bei Überschreitung des jeweiligen Grenzwertes ausgegeben und/oder in dem Speicher abgelegt. So kann beispielsweise für einen mechanischen oder Stoss-Sensor eine Maximalgröße einer auf das Instrument einwirkenden Kraft vorgegeben werden, wobei bei Überschreitung dieser Kraft zum Beispiel angenommen wird, dass die Kalibrierung des Instrumentes verloren gegangen ist und dieses noch mal kalibriert werden sollte. Hierzu kann auch eine Warnmeldung entweder unmittelbar bei Auftreten der externen Einwirkung oder auch zeitverzögert, zum Beispiel nach Abfrage des Kalibrier-Zustandes, ausgegeben werden. Im Falle der Verwendung von Temperatursensoren kann zum Beispiel eine minimale und eine maximale Temperatur vorgegeben, wobei bei Unter- oder Überschreitung dieser Temperaturen ebenfalls entsprechende Warnmeldungen ausgegeben oder abgespeichert werden können.

Weiterhin betrifft die Erfindung ein Computerprogramm, welches ein wie oben beschriebenes Verfahren ausführen oder unterstützen kann und ein Programmspeichermedium mit einem solchen Programm.

Die Erfindung wird nachfolgend anhand von Ausführungsbeispielen beschrieben werden. Es zeigen:
- Figur 1: Ein erfindungsgemäßes Navigationssystem; und
- Figur 2: Ein Ablaufdiagram zur Erläuterung des erfindungsgemäßen Verfahrens.

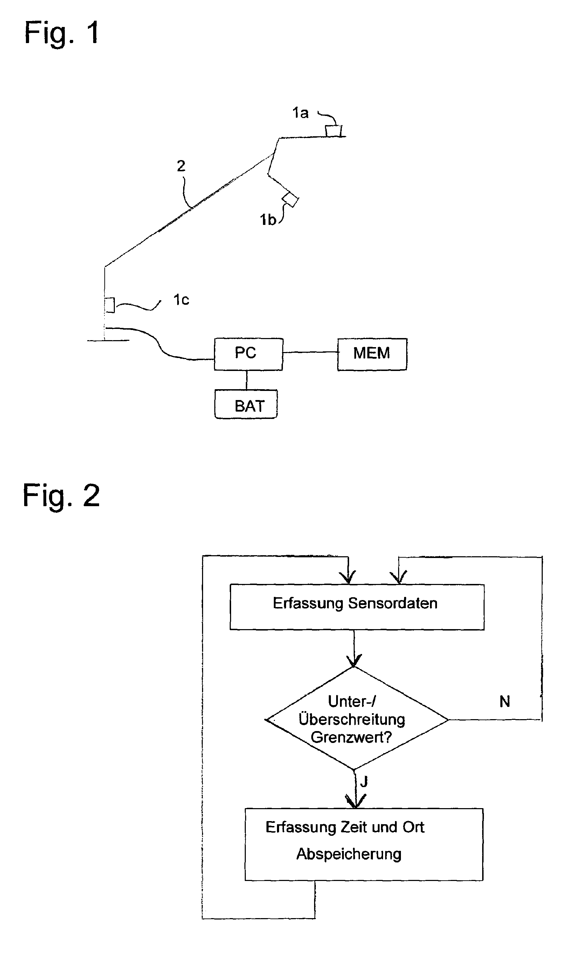

Figur 1 zeigt eine präkalibrierte IR-Kamera 2, an welcher an den Positionen der Kameras jeweils ein Stosssensor 1a und 1b vorgesehen ist. Weiterhin ist im Stativ oder der IR-Kamera 2 eine aus mehreren Sensoren 1c bestehende Anordnung vorgesehen, mit welcher mechanische Stöße, die Temperatur und weitere Größen erfasst werden können. Diese Sensoren 1a bis 1c sind über einen Rechner PC mit einem Speicher MEM verbunden, welche mit einer Batterie BAT verbunden sind, welche die Stromversorgung für die Sensoren 1a bis 1c und die damit verbundenen Einheiten sicherstellt. Tritt zum Beispiel während des Transportes des Navigationssystemes ein Stoss auf, da dieses zum Beispiel gegen eine Aufzugtür geschoben wurde, so wird in dem Speicher MEM ein Logbuch-Eintrag hinterlegt, in welchem die Stärke des Stoßes zusammen mit der Uhrzeit, bei welcher der Stoß aufgetreten ist, und dem zugehörigen GPS-Signal abgespeichert wird. Wird das System in Betrieb genommen, liest der Rechner PC, der auch ein Mikrokontroller sein kann, den Speicher MEM aus und gibt eine Warnung aus, dass ein starker mechanischer Stoß detektiert wurde, welcher möglicherweise die Kalibrierung des Systems verändert hat, so dass dieses System neu kalibriert werden sollte. Ebenso können mittels der Sensoranordnung 1c der Umgebungsdruck zum Beispiel während eines Transportes per Flugzeug, Feuchtigkeitsschwankungen, oder Strahlenbelastungen des Systems aufgezeichnet werden, welche Pixel-Artefakte verursachen können, die zu einer Beschädigung des Tracking-Systems oder zu einer Verringerung der Genauigkeit führen. Ebenso kann detektiert werden, ob zum Beispiel während eines Reinigungsvorganges Flüssigkeit in das System eingetreten ist oder ob das System extremen magnetischen Feldern ausgesetzt wurde, was zum Beispiel in einer MRT-Umgebung der Fall sein kann.

Wie in Figur 2 gezeigt, werden die Daten der Sensoren 1a bis 1c bevorzugt kontinuierlich erfasst, wobei überprüft wird, ob ein vorgegebener Grenzwert unter- oder überschritten wird. Falls keine Unter- oder Überschreitung des Grenzwertes dektiert wurde, wird die Erfassung der Sensordaten fortgesetzt. Falls eine Unter- oder Überschreitung vorliegt, so werden zusätzlich zu dem Sensorsignal Uhrzeit und Ort bzw. GPS-Signal erfasst und zusammen mit dem Sensorsignal in dem Speicher MEM abgelegt, wo sie später bei oder nach der Inbetriebnahme des Systems wieder ausgelesen werden können.

## Patentansprüche

1. Kalibriertes Instrument mit mindestens einem Sensor (1a, 1b, 1c), um Umwelteinflüsse zu detektieren, welche die Kalibrierung oder Funktionsfähigkeit des Instrumentes (2) verändern können.

2. Instrument nach Anspruch 1, wobei das Instrument ein Tracking-System, die Kamera (2) eines Tracking-Systems, eine Instrumentekalibrier-Matrix, ein Fluoro-Registrierungs-Kit, ein Pointer, ein Ultraschallphantom, eine MR-Spulenanordnung, insbesondere Kopfspulen, oder ein Implantat ist.

3. Instrument nach einem der vorhergehenden Ansprüche, wobei der mindestens eine Sensor (1a-c) ein Temperatur-Sensor, Stoss- oder Schock-Sensor, ein Beschleunigungssensor, ein Dehnungssensor, ein Feuchtigkeitssensor, ein Magnetfeldsensor, ein Drucksensor und/oder ein Strahlungssensor ist.

4. Instrument nach einem der vorhergehenden Ansprüche mit einem Speicher (MEM), welcher mit dem Sensor (1a-c) verbunden ist.

5. Instrument nach einem der vorhergehenden Ansprüche mit einer Energiequelle, insbesondere einer Batterie, welche mit dem Sensor (1a-c) verbunden ist und/oder mit Sensoren, welche keine externe Energiequelle benötigen.

6. Instrument nach einem der vorhergehenden Ansprüche mit einem Timer und/oder einem. Ortserfassungs- oder GPS-System.

7. Instrument nach einem der vorhergehenden Ansprüche mit einer Statusanzeige zur Ausgabe eines Signals, welches angibt, ob das Instrument kalibriert werden soll.

8. Instrument nach einem der vorhergehenden Ansprüche, wobei ein abnehmbarer Datenträger vorgesehen ist, in welchem Sensorsignale abgespeichert werden können.

9. Instrument nach einem der vorhergehenden Ansprüche mit einer Schnittstelle, welche mit mindestens einem Sensor und/oder einem Speicher (MEM) und/oder einem Timer und/oder Ortserfassungssystem verbunden ist, um die korrespondierenden Daten für eine Fernwartung verfügbar zu machen.

10. Verfahren zum Detektieren mindestens eines externen Einflusses auf ein kalibriertes Instrument (2), wobei mindestens eine externe physikalische oder chemische Größe erfasst und überprüft wird, ob diese Größe die Kalibrierung des Instrumentes (2) verändern kann.

11. Verfahren nach dem vorhergehenden Anspruch, wobei die Erfassung der externen Größe kontinuierlich durchgeführt wird.

12. Verfahren nach einem der zwei vorhergehenden Ansprüche, wobei das erfasste Signal kontinuierlich oder nach Unter- oder Überschreitung eines Grenzwertes aufgezeichnet wird.

13. Verfahren nach einem der drei vorhergehenden Ansprüche, wobei zusätzlich zu dem Signal zur Erfassung der externen Größe ein Zeit- und/oder Ortsignal erfasst wird.

14. Verfahren nach einem der vier vorhergehenden Ansprüche, wobei ein Warnsignal ausgegeben oder zum Abruf bereitgestellt wird, wenn das Instrument (2) kalibriert werden soll.

15. Verfahren nach einem der fünf vorhergehenden Ansprüche, wobei die erfasste mindestens eine externe physikalische oder chemische Größe in einem abnehmbaren Datenträger abgespeichert und/oder über eine Schnittstellte zur Fernwartung verfügbar gemacht wird.

16. Computerprogramm welches, wenn es in einen Computer geladen wird oder auf diesem läuft, ein Verfahren nach einem der fünf vorhergehenden Ansprüche ausführt oder unterstützt.

17. Programmspeichermedium oder Computerprogrammprodukt mit dem Computerprogramm nach dem vorhergehenden Anspruch.
